# EUROPÄISCHE PATENTSCHRIFT

(11) **EP 1 450 773 B1**
(45) Veröffentlichungstag und Bekanntmachung des Hinweises auf die Patenterteilung: **28.05.2008**
(21) Anmeldenummer: 02804178.8
(22) Anmeldetag: 16.11.2002
(51) Int. Cl.: A61K 9/70

(54) **TRANSDERMALES THERAPEUTISCHES SYSTEM MIT VERBESSERTEM LANGZEITTRAGEKOMFORT**
TRANSDERMAL THERAPEUTIC SYSTEM PROVIDED WITH IMPROVED LONG-TERM CARRYING COMFORT
SYSTEME THERAPEUTIQUE TRANSDERMIQUE A CONFORT DE PORT LONGUE DUREE AMELIORE

(30) Priorität: 05.12.2001 DE 10159745
(43) Veröffentlichungstag der Anmeldung: 01.09.2004
(73) Patentinhaber: LTS LOHMANN Therapie-Systeme AG, 56626 Andernach (DE)
(72) Erfinder: HILLE, Thomas, 56567 Neuwied (DE); DEURER, Lothar, 56077 Koblenz (DE)
(74) Vertreter: Schmidt, Werner
(86) Internationale Anmeldenummer: PCT/EP2002/012875
(87) Internationale Veröffentlichungsnummer: WO 2003/047556

(56) Entgegenhaltungen:
- EP-A- 0 274 863
- EP-A- 0 427 877
- WO-A-90/06736
- DE-A- 10 103 860
- DE-A- 19 738 855
- DE-A- 19 923 551
- DE-C- 10 027 258
- US-A- 5 246 705
- ROZENBAUM HENRI ET AL: "Comparison of two estradiol transdermal systems (Oesclim 50 and Estraderm TTS 50). I. Tolerability, adhesion and efficacy." MATURITAS, Bd. 25, Nr. 3, 1996, Seiten 161-173, XP002230890 ISSN: 0378-5122
- TAURELLE R ET AL: "The long-term tolerability and efficacy of OESCLIM: results of a 1-year study." MATURITAS. IRELAND NOV 1999, Bd. 33 Suppl 1, November 1999 (1999-11), Seiten S73-S81, XP002230989 ISSN: 0378-5122
- MUNOZ A: "OESCLIM: an advanced delivery system for HRT." MATURITAS. IRELAND NOV 1999, Bd. 33 Suppl 1, November 1999 (1999-11), Seiten S39-S47, XP002230986 ISSN: 0378-5122

## Beschreibung

Die Erfindung bezieht sich auf ein Transdermales Therapeutisches System (TTS) für die kontrollierte Verabreichung eines pharmazeutischen Wirkstoffs. Das TTS ist für eine längere, insbesondere mehrtägige Verabreichung des Wirkstoffs geeignet und ermöglicht einen kontinuierlichen Kontakt mit der Hautoberfläche eines Anwenders auch während eines Zeitabschnitts, der durch ein intensives Ausgesetztsein des auf der Hautoberfläche angebrachten TTS mit Wasser gekennzeichnet ist, ohne dass ein unerwünschtes Ablösen auftritt.

Transdermale Therapeutische Systeme (TTS) sind schichtförmig aufgebaute Pflaster, die mindestens einen pharmazeutischen Wirkstoff in einer Reservoirschicht enthalten. Man unterscheidet Matrix- bzw. Reservoirtyp-TTS, wobei im ersten Fall die den pharmazeutischen Wirkstoff enthaltende Reservoirschicht haftklebend ist und im zweiten Fall eine die Geschwindigkeit der Freisetzung des pharmazeutischen Wirkstoffs kontrollierende Membran und ggf. eine zusätzliche Haftkleberschicht vorhanden sind.

Die meisten der kommerziell erhältlichen TTS sind für eine 24-stündige Anwendung konzipiert. Es gibt jedoch auch so genannte Dreitagespflaster, die vom Anwender (z. B. eine Person, die die kontinuierliche Verabreichung des pharmazeutischen Wirkstoff über einen längeren Zeitraum benötigt, Patient, etc.) über einen Zeitraum von mindestens 72 Stunden getragen werden sollen, z. B. Duragesic. Der vorgesehene Anwendungszeitraum ist eine typische Kenngröße für ein spezielles Transdermales Therapeutisches System und jeweils den Anwenderinstruktionen in der Packungsbeilage zu entnehmen.

Die Funktionsfähigkeit eines TTS beruht darauf, dass die Konzentration des pharmazeutischen Wirkstoffs in der Reservoirschicht deutlich höher ist als in den Blutgefäßen, die unterhalb der Hautoberfläche des Anwenders liegen, auf die das Transdermale Therapeutische System angebracht ist (Applikationsort). Aufgrund dieses Konzentrationsgefälles entsteht eine gerichtete Diffusion, die nach einer kurzen Anfangsphase (der so genannten "lag time") für einen weitgehend konstanten Wirkstofffluss sorgt.

Dabei bildet sich zwischen der wirkstoffhaltigen Reservoirschicht des TTS, der gegebenenfalls vorhandenen Membran, der gegebenenfalls vorhandenen Haftkleberschicht, den Hautschichten, die unter der Hautoberfläche des Anwenders liegen, auf der das TTS angebracht ist und dem Blutkreislauf ein Fließgleichgewicht. Eine Unterbrechung dieses Fließgleichgewichts - zum Beispiel durch ein kurzzeitiges Entfernen des TTS - kann dazu führen, dass die Verhältnisse des sich nach der Anlaufphase eingestellten Fließgleichgewichts erheblich verändert werden und eine kontinuierliche und konstante Verabreichung des pharmazeutischen Wirkstoffs nicht gewährleistet werden kann.

Probleme können bei der Langzeitapplikation von TTS auch dadurch auftreten, dass ein Patient wegen der Starrheit des Systems, insbesondere der Rückschicht, ein ausgeprägtes Fremdkörpergefühl entwickeln. Außerdem kann diese Starrheit einer Komponente (wirkstoffhaltige Matrix, Rückschicht etc.) ein vorzeitiges Ablösen bis hin zum Abfallen des TTS von der Haut bewirken, weil für ein ausreichendes Haften des TTS nicht nur gute Klebeeigenschaften, sondern auch eine hinreichende Flexibilität des TTS erforderlich ist, da ein TTS sich den Bewegungen der Haut anpassen sollte.

Ferner führt das Tragen eines TTS fast immer - insbesondere bei einem mehr als 24-stündigen Tragen - zur Okklusion am Applikationsort, da die Haut an dieser Stelle den Schweiß nur begrenzt abgeben kann. Dies führt einerseits zur Hydratation der Haut, andererseits wird das Wachstum von Mikroorganismen im feucht-warmen Mikroklima begünstigt.

Auch kann der durch die Verabreichung des pharmazeutischen Wirkstoffs auftretende Verlust des Wirkstoffs in der Reservoirschicht die Zusammensetzung dieser Schicht erheblich verändern. Dies kann eine Rolle spielen, wenn der pharmazeutische Wirkstoff als Weichmacher für das Polymermaterial wirken kann, aus welchem die Reservoirschicht aufgebaut ist. Solche Effekte können gegebenenfalls durch Zugabe von anderen Weichmachern und / oder Veränderung des Polymerisations- und / oder Vernetzungsgrads des für den Aufbau der Reservoirschicht verwendeten Polymermaterials kompensiert werden.

Bei einer Anwendung eines TTS über einen längeren Zeitraum (mindestens 24 Stunden) kann auch das Problem auftreten, dass größere Mengen Feuchtigkeit an das TTS gelangen und eine Zersetzung der Bestandteile der Reservoirschicht und / oder ein unerwünschtes Ablösen von der Hautoberfläche bewirken.

Als Feuchtigkeit kommen hier einerseits Schwitzwasser - aus den unter dem Applikationsort des TTS liegenden Schweißdrüsen der Haut des Anwenders abgesondertes Wasser - und Wasser, welches zum Beispiel beim Waschen (Baden, Duschen, Sauna etc.) an die Außenseite des auf der Hautoberfläche des Anwenders angebrachten TTS gelangen kann, in Frage.

Dem Schwitzwasser ist das TTS über den gesamten Anwendungszeitraum ausgesetzt, wobei die Gesamtmenge eine verhältnismäßig geringe, aber kontinuierlich existierende Belastung für das TTS darstellt. Beim Baden, Duschen etc. ist dagegen das TTS für einen bestimmten Zeitabschnitt von außen herantretendem Wasser ausgesetzt, dafür aber mit erheblich größerer Intensität.

Das US-Patent 4,911,916 offenbart ein Transdermales Therapeutisches System (TTS) mit einer elastischen Rückschicht, einer Diffusionsmatrix aus einem netzförmigen, makroporösen Polymerschaum und einer hydrophoben Haftkleberschicht. In den Poren der makroporösen Diffusionsmatrix ist ein viskoelastisches Polymer enthalten, welches einen zumindest teilweise gelösten Wirkstoff enthält. Die Rückschicht wird so gewählt, daß sie die Okklusivität des TTS gewährleistet. Als Vorteil wird hierdurch die Erhöhung der Permeationsrate des Wirkstoffs erreicht.

Aus EP 274 863 A2 ist ein wasserdampfdurchlässiger Verbandstoff zur kontrollierten Verabreichung von topischen Medikamenten an die Haut bekannt. Der Verbandstoff besitzt ein Reservoir aus einer Polyvinylchloridschicht, die aus einem Polyvinylchloridharz und einem primären Weichmacher für das Polyvinylchloridharz hergestellt wird. Bei den in dem Reservoir enthaltenen topischen Medikamenten handelt es sich um solche, die keine systemische Wirkung besitzen, sondern ausschließlich an der Kontaktstelle des Verbandstoffs zum betroffenen Gewebe des Patienten eine äußerliche, insbesondere eine antibakterielle oder desinfizierende Wirkung erzielen.

Das US-Patent 5,246,705 offenbart ein Transdermales Therapeutisches System mit einer dehnbaren, okklusiven Rückschicht. Die Materialien dieser Rückschicht sind resistent gegenüber Flüssigkeit uns besitzen eine Wasserdampfdurchlässigkeit im Bereich von 0,1 bis 20 g/m²/hr, vorzugsweise zwischen 0,1 und 2 g/m²/hr.

In der WO 90/06736 A wird ein hormonhaltiges TTS mit einer Rückschicht offenbart, die atmungsaktiv ist und eine Mikroporosität besitzt. In dem Dokument wird jedoch nicht erläutert, wie geforderte Mikroporosität der Rückschicht erreicht wird.

EP 427 877 B1 offenbart einen Clonidin enthaltenden Pflasterverband mit einer porösen Polytetrafluorethylenschicht. Diese Schicht besitzt eine bestimmte Luftdurchlässigkeit, die durch das spezielle Herstellverfahren mit ein- oder zweiachsigem Strecken unter gleichzeitigem Sintern erreicht werden kann.

Es ist eine Aufgabe der Erfindung, einen pharmazeutischen Wirkstoff über einen längeren Zeitraum an eine Person zu verabreichen, die eine kontrollierte und kontinuierliche Verabreichung dieses Wirkstoffs über einen längeren Zeitraum benötigt.

Eine weitere Aufgabe ist es, ein Transdermales Therapeutisches System (TTS) zur Verfügung zu stellen, das ein kontinuierliches Tragen dieses TTS auf einer bestimmten Stelle der Hautoberfläche (Applikationsort) über einen längeren Zeitraum ermöglicht und während dieses Zeitraums eine kontrollierte und kontinuierliche Verabreichung dieses Wirkstoffs gewährleistet.

Eine weitere Aufgabe ist es, ein unerwünschtes Ablösen dieses TTS während des längeren Zeitraums zu verhindern, selbst wenn innerhalb dieses Zeitraums mindestens ein Zeitabschnitt enthalten ist, in dem das Transdermale Therapeutische System einem intensiven Kontakt mit Wasser ausgesetzt ist.

Eine weitere Aufgabe ist es, eine Klebermatrix für ein Transdermales Therapeutisches System (TTS) zur Verfügung zu stellen, die das kontinuierliche Tragen dieses TTS über einen längeren Zeitraum ermöglicht, insbesondere unter den Bedingungen, dass innerhalb dieses Zeitraums mindestens ein Zeitabschnitt enthalten ist, in dem das Transdermale Therapeutische System einem intensiven Kontakt mit Wasser ausgesetzt ist.

Eine weitere Aufgabe ist es, eine TTS-Konstruktion zur Verfügung zu stellen, die ein Abdiffundieren von Schwitzwasser (Transpiration, Verdunsten von Schweiß) durch die verschiedenen Schichten des TTS ermöglicht.

Die Aufgabe wird gelöst durch ein Verfahren, bei dem ein Transdermales Therapeutisches System (TTS), welches eine mindestens einen pharmazeutische Wirkstoff enthaltende Reservoirschicht aufweist, über einen längeren Zeitraum auf der Hautoberfläche eines Anwenders angebracht wird, dort (an dem speziellen Applikationsort) kontinuierlich haftet und den pharmazeutischen Wirkstoff transdermal abgibt. Das hierbei verwendete Transdermale Therapeutische System ist ebenfalls ein Teil der Lösung der Aufgabe.

Der längere Zeitraum umfasst die Zeitspanne zwischen dem Anbringen eines Transdermalen Therapeutischen Systems auf der Hautoberfläche eines Anwenders und dem Abziehen dieses Transdermalen Therapeutischen Systems nach Ablauf des vorgesehenen Anwendungszeitraums. Der längere Zeitraum ist ein Zeitraum von mindestens 24 Stunden, vorzugsweise von mindestens 48 Stunden und besonders bevorzugt ein Zeitraum von mindestens 72 Stunden. In einer weiteren besonders bevorzugten Ausführungsform umfasst der längere Zeitraum mindestens etwa 168 Stunden. Es versteht sich von selbst, dass während des längeren Zeitraums eine Abgabe von Schwitzwasser (Schweiß) von der Haut erfolgt. Der längere Zeitraum entspricht dem Anwendungszeitraum eines einzelnen speziellen TTS.

Der vorgesehene Anwendungszeitraum ist eine bestimmte Kenngröße für ein Transdermales Therapeutisches System, der üblicherweise dem Beipackzettel zu entnehmen ist. Innerhalb des Anwendungszeitraums ist die Wirkstoffabgabe durch das TTS weitgehend konstant. Der längere Zeitraum kann mindestens einen Zeitabschnitt intensiven Ausgesetztseins des auf der Hautoberfläche haftenden TTS mit Wasser umfassen.

Unter einem Zeitabschnitt intensiven Ausgesetztseins des auf der Hautoberfläche haftenden TTS mit Wasser ist zum Beispiel der Fall eines Bades, eines Duschvorgangs oder eines Saunabesuchs zu verstehen. Ein solcher Zeitabschnitt ist natürlich individuell vom Anwender abhängig, wobei jedoch in gewissen Kulturkreisen die Dauer und das Ausmaß der Körperreinigung eine längere Wasserkontaktzeit mit sich bringt. Üblicherweise können solche Zeitabschnitte eine Dauer von mindestens 1 Minute bis maximal 30 Minuten umfassen, wobei von einer durchschnittlichen Dauer von 5 bis 15 Minuten auszugehen ist.

Der Begriff kontinuierlich im Sinne dieser Beschreibung bedeutet, dass der Kontakt zwischen der Hautoberfläche des Anwenders, auf der das TTS angebracht wurde (d. h. dem Applikationsort) und der Seite des Transdermalen Therapeutischen Systems, aus der der mindestens eine pharmazeutische Wirkstoff an die Hautoberfläche des Anwenders abgegeben wird, während des gesamten längeren Zeitraums nicht unterbrochen wird. Das bedeutet, dass während des Zeitraums zwischen dem Anbringen eines Transdermalen Therapeutischen Systems am speziellen Applikationsort auf der Hautoberfläche eines Anwenders und dem Abziehen dieses Transdermalen Therapeutischen Systems nach Ablauf des vorgesehenen Anwendungszeitraums kein unerwünschtes Ablösen von dieser Hautoberfläche erfolgt.

Der Begriff kontinuierlich ist in dem Zusammenhang mit dem Haften auf der Hautoberfläche nicht streng gleichzusetzen mit einer kontinuierlichen Verabreichung des pharmazeutischen Wirkstoffs an den Anwender, da üblicherweise - wie oben beschrieben - erst ein Fließgleichgewicht zwischen der wirkstoffhaltigen Reservoirschicht, den Hautschichten und den Blutgefäßen des Anwenders entstehen muss. Diese diffusionsbedingten Erscheinungen bewirken, dass während einer so genannten lag-time kein bzw. nur eine geringe Menge des mindestens einen pharmazeutischen Wirkstoffs an die Haut abgegeben wird. Diese lag-time ist im allgemeinen jedoch in Hinblick auf die gesamte Zeitspanne zwischen dem Applizieren auf der Hautoberfläche und dem Abziehen des Transdermalen Therapeutischen Systems, d. h. dem vorgesehenen Applikationszeitraums vernachlässigbar kurz. Die lag-time spielt im übrigen nur bei der erstmaligen Anwendung eines TTS eine Rolle, da im Rahmen einer transdermalen Therapie üblicherweise mehrere TTS in zeitlich aufeinanderfolgender Reihe appliziert werden.

Unter dem Begriff transdermal ist die perkutane Abgabe mindestens eines pharmazeutischen Wirkstoffs an einen Anwender (Patienten) zu verstehen, wobei mindestens ein pharmazeutischer Wirkstoff aus einem Transdermalen Therapeutischen System an die Hautoberfläche des Anwenders abgegeben wird und durch die verschiedenen, unter dem Applikationsort des TTS liegenden Hautschichten (z. B. stratum comeum, dermis, cutis und subcutis) wandert, bis er von den darunter liegenden Blutgefäßen aufgenommen wird.

Das Transdermale Therapeutische System (TTS), welches im Rahmen des genannten Verfahrens eingesetzt werden kann, umfasst eine Rückschicht, eine den mindestens einen pharmazeutischen Wirkstoff enthaltende Reservoirschicht und ein Klebemittel. Die Rückschicht ist flexibel und durchlässig für Wasserdampf, jedoch für Wasser in flüssiger Form undurchlässig. Sie liegt in Form einer perforierten Folienbahn vor und weist zwischen 200 und 2.500 Poren pro cm² auf, welche einen Durchmesser zwischen 2 und 150 µm besitzen. Während der Lagerung befindet sich ein solches TTS üblicherweise auf einer wiederablösbaren Schutzschicht, von der es unmittelbar vor der Anbringung auf der Hautoberfläche des Anwenders abgezogen wird. Gelagert wird ein solchermaßen geschütztes TTS in einer Blisterverpackung oder einem Siegelrandbeutel.

Die Rückschicht befindet sich auf der hautabgewandten Seite des TTS. Sie liegt in Form einer Folienbahn oder einer Kombination einer Folienbahn mit einem Gewebe vor. Die Rückschicht ist vorzugsweise flexibel, worunter die Fähigkeit verstanden wird, sich bei Einwirkung einer senkrecht auf die Schicht weisenden geringen Kraft leicht zu biegen.

Unter einer Folienbahn ist ein flächiges Material zu verstehen, das vorzugsweise flexibel ist. Die Dicke einer solchen Folienbahn kann zwischen 2 und 50 µm liegen, vorzugsweise zwischen 5 und 36 µm und besonders bevorzugt zwischen 9 und 23 µm. Die Folienbahn kann aus Kunststoff oder Metall bestehen oder mindestens eines dieser Materialien in einem Verbundlaminat enthalten. Die Folienbahn kann transparent oder undurchsichtig sein.

Als ein Kunststoff, aus dem die Rückschicht bestehen kann, kommt grundsätzlich jeder synthetisch herstellbare Polymerstoff in Frage, wobei jedoch Polyester, vorzugsweise Polyethylenterephthalat bevorzugt wird. Aber auch Polyurethane und Polyether/ester-Elastomere (Blockpolymere aus teilkristallinem Polybutylenterephthalat und Polyetherdiolen bzw. langkettigen aliphatischen Dicarbonsäureestern) sind hierfür besonders geeignet. Als Metall kommt insbesondere Aluminium in Frage.

Als Verbundlaminat können Folienbahnen eingesetzt werden, die durch Verleimen, Laminieren, Kaschieren oder Extrudieren aus mindestens einem ersten Kunststoff und einem weiteren Kunststoff oder Metall erhalten werden.

Die Folienbahn ist perforiert, wobei die Poren einen Durchmesser zwischen 2 µm und 150 µm annehmen. Die Porendichte liegt zwischen 200 bis 2.500 Poren pro cm², vorzugsweise zwischen 500 und 1.700. Eine bevorzugte Porenform besitzt eine trichterförmige Lochstruktur, was spezifische Kapillareigenschaften zur Folge hat. Während die "glatte" Seite einer solchen Folienbahn einen freien Durchfluß von Wasser gewährleistet, wirkt die rauhe Seite als Sperre. Eine perforierte Folienbahn mit trichterförmiger Lochstruktur als Rückschicht ist daher in dem TTS so angeordnet, daß ihre rauhe Seite zur hautabgewandten Seite weist.

Unter einem Gewebe ist ein Webereierzeugnis zu verstehen, welches üblicherweise aus Kettfäden und Schussfäden hergestellt wird. Das fadenförmige Ausgangsmaterial eines Gewebes kann aus natürlichen oder synthetischen Polymerstoffen bestehen. Als natürlicher Polymerstoff kann z. B. Cellulose, Seide und Baumwolle, als synthetischer Polymerstoff kann z. B. Polyvinylchlorid, Polyurethan, Polyester, Polyamid, Nomex, Kevlar, Polypropylen, Polyacryl, Preox, Trevira, Nylon oder Kunstseide in Frage kommen.

Die Rückschicht ist wirkstoffundurchlässig, womit gemeint ist, dass die Rückschicht befähigt ist, ein Austreten des pharmazeutischen Wirkstoffs durch die Rückschicht zu verhindern. Die Rückschicht ist feuchtigkeitsdurchlässig, wobei darunter zu verstehen ist, dass die Rückschicht durchlässig für Wasserdampf ist. Die Rückschicht ist für Wasser in flüssiger Form undurchlässig.

Die Rückschicht kann auch elastisch sein, was für den Tragekomfort eine große Bedeutung besitzt. Hierunter ist zu verstehen, dass die Rückschicht bei Anwendung einer äußeren Zugkraft zu einer Streckung befähigt ist, die bei Wegfall dieser Zugkraft ebenfalls entfällt. Die Rückschicht nimmt dann wieder die ursprünglichen Dimensionen an. Diese Befähigung zur reversiblen Veränderung der Dimensionen ist in mindestens einer Richtung ausgestaltet und beträgt mindestens 10%, vorzugsweise mindestens 30%. In einer besonders bevorzugten Ausführungsform ist die Rückschicht elastisch und wasserdampfdurchlässig.

Die Reservoirschicht enthält den mindestens einen pharmazeutischen Wirkstoff sowie mindestens ein polymeres Trägermaterial. Der Gehalt des mindestens einen pharmazeutischen Wirkstoffs in der Reservoirschicht liegt zwischen 0,5 und 45 Gew.-%, vorzugsweise zwischen 3 und 25 Gew.-%. Der Gehalt des mindestens einen pharmazeutischen Wirkstoffs in der Reservoirschicht ist ausreichend, dass der Wirkstoff während des längeren Zeitraum kontinuierlich an die Hautoberfläche abgegeben wird. Weiterhin ist der Gehalt des mindestens einen pharmazeutischen Wirkstoffs ausreichend, dass durch den mindestens einen Zeitabschnitt intensiven Ausgesetztseins des auf der Hautoberfläche haftenden TTS mit Wasser (innerhalb des längeren Zeitraums) die Haftung des TTS nicht unterbrochen wird, d. h. es erfolgt kein Ablösen des TTS.

Als pharmazeutischer Wirkstoff kommen Abmagerungsmittel, Appetitzügler, Acidosetherapeutika, Alzheimermittel, Analeptika, Antihypoxämika, Analgetika, Antirheumatika, Anthelminthika, Antiallergika, Antianämika, Antiarrhythmika, Antibiotika, Antiinfektiva, Antidementiva (Nootropika), Antidiabetika, Antidota, Antieetika, Antivertiginosa, Antiepileptika, Antihämorrhagika (Antifibrinolytika), Antihypertonika, Antihypoglykämika, Antihypotonika, Antikoagulantia, Antimykotika, antiparasitäre Mittel, Antiphlogistika, Antitussiva, Expektorantia, Arteriosklerosemittel, Balneotherapeutika und Mittel zur Wärmetherapie, Betarezeptorenblocker, Calciumkanalblocker, Hemmstoffe des Renin-Angiotensin-Systems, Broncholytika, Antiasthmatika, Cholagoga, Gallenwegstherapeutika, Cholinergika, Corticoide, Dermatika, Desinfizientia, Antiseptika, Diätetika, Emährungstherapeutika, Diuretika, durchblutungsfördernde Mittel, Entwöhnungsmittel, Enzyminhibitoren, Enzympräparate, Transportproteine, Fibrinolytika, Geriatrika, Gichtmittel, Mittel gegen grippale Infekte und Erkältungskrankheiten (Grippemittel), Gynäkologika, Hämorrhoidenmittel (Proktologika), Hepatika, Hypnotika, Sedativa, Hypophysenhormone, Hypothalamushormone, regulatorische Peptide und ihre Hemmstoffe, Immuntherapeutika, Zytokine, Kardiaka, Kariesmittel, Paradontosemittel, Koronarmittel, Laxantia, Lipidsenker, Lokalanäathetika, Neuraltherapeutika, Magen-Darm-Mittel, Migränemittel, Mineralstoffe, Muskelrelaxantia, Narkosemittel, Nebenschilddrüsenhormone, Calciumstoffwechselregulatoren, Osteoporosemittel, Neuropathiepräparate, neurotrope Mittel, Ophthalmika, Otologika, Parkinsonmittel, Mittel gegen extrapyramidale Störungen, Psychopharmaka, Rhinologika, Sinusitismittel, Roborantia, Tonika, Schilddrüsentherapeutika, Sera, Immunglobuline und Impfstoffe, Sexualhormone und ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika und Metastasenhemmer in Frage.

Hierzu zählen neben anderen auch insbesondere: 17β-Estradiol, Norethisteron, Physostigmin, Norelgestromin, Norethisteronacetat, Nitroglycerin, Nicotin, Clonidin, Moxonidin, Fentanyl, Testosteron, Buprenorphin, Galanthamin, Rivastigmin, Morpin, Diamorphin, Buprion, Sildenafil, (-)-5,6,7,8,-Tetrahydro-6-[propyl[2-(2-thienyl)-ethyl]amino]-1-naphthol, Methylphenidat, Ethinylestradiol und (S)-N-ethyl-3-[1-dimethylamino)ethyl]-N-methyl-phenyl-carbamat.

Der mindestens eine pharmazeutische Wirkstoff ist in der Reservoirschicht des TTS in einer solchen Menge enthalten, die ausreichend ist, dass über den vorgesehenen längeren Zeitraum (Anwendungszeitraum) der mindestens eine pharmazeutische Wirkstoff in einer wirksamen Menge an den Blutkreislauf abgegeben wird. Hierunter ist zu verstehen, dass gegebenenfalls eine Anfangsphase auftreten kann, während der die Wirkstoffabgabe nicht der weitgehend konstanten Wirkstoffabgabe während des überwiegenden Teils des längeren Zeitraums entspricht ("lag-time").

Das polymere Trägermaterial bildet einen wesentlichen Bestandteil der Reservoirschicht. Es können in Frage kommen: wasserabweisende Polymere, wasserquellbare Polymere, wasserlösliche Polymere, wasserdampfpermeable Polymere, die dem Fachmann bekannt sind.

Die Unterseite dieser Reservoirschicht kann mit der Seite des Transdermalen Therapeutischen Systems identisch sein, aus der der pharmazeutische Wirkstoff an die Hautoberfläche des Anwenders abgegeben wird ("Hautseite" = die den Wirkstoff freisetzende Seite des TTS). Sofern jedoch eine die Geschwindigkeit der Freisetzung des Wirkstoffs kontrollierende Membran und / oder eine zusätzliche Haftkleberschicht vorhanden sind, die unterhalb der Reservoirschicht angeordnet sind, bilden diese dementsprechend diese Seite des TTS.

Als Klebemittel kann eine Komponente der Reservoirschicht sein, die bewirkt, dass diese dadurch haftklebend wird. Das Klebemittel kann auch eine separate Haftkleberschicht, die auf der zur Haut weisenden Seite des TTS angebracht ist, oder ein Überpflaster sein.

Als Komponente der Reservoirschicht, die bewirkt, dass diese Schicht haftklebend wird, kann ein Klebrigmacher ("tackifier") verwendet werden. Geeignete Klebrigmacher sind beispielsweise Abietylalkohol und seine Derivate, z. B. Abietylester.

Auch kann die Beschaffenheit der polymeren Trägermaterials der Reservoirschicht derart sein, dass diese Schicht "haftklebend" ist. In diesem Fall werden Monomere, die als Bestandteil des polymeren Trägermaterials zum Vorliegen haftklebender Eigenschaften beitragen, bei der Herstellung des polymeren Trägermaterials verwendet. Geeignete Monomere sind: Polysiloxane, Polyisobutylene und Polyacrylate, vorzugsweise solche aus Acrylsäure und / oder Methylacrylsäure und / oder ihren Ester, z. B. Isooctylacrylat, 2-Ethylhexylacrylat etc. Als haftklebend wird die Eigenschaft bezeichnet, dass ein Material in trockener Form und bei Raumtemperatur eine permanente Anfangsklebkraft ("tack") besitzt, die dazu befähigt, dass das Material auf einer Vielzahl unterschiedlicher Materialien fest haftet, ohne dass dazu ein Anpressdruck benötigt wird, der größer ist als der mit einem Finger auszuüben ist.

In einer weiteren Ausführungsform kann eine zusätzliche Haftkleberschicht verwendet werden, die mit einem höheren Anteil von Klebrigmachern versehen ist. In einer weiteren Ausführungsform kann ein Überpflaster verwendet werden. In diesem Fall wird die den mindestens einen pharmazeutischen Wirkstoff enthaltende Reservoirschicht mit einem Verbund aus Rückschicht und Haftkleberschicht soweit überzogen, dass ein überstehender Rand entsteht. Die Haftkleberschicht des überstehenden Rands ist dazu befähigt, den kontinuierlichen Kontakt zwischen Hautoberfläche und Reservoirschicht sicherzustellen.

Die erfindungsgemäße Lösung besteht in dem Verfahren, bei dem an einen Anwender (Patienten), der die kontinuierliche Verabreichung dieses pharmazeutischen Wirkstoffs über einen längeren Zeitraum benötigt, dieser mindestens eine pharmazeutische Wirkstoff an den Anwender verabreicht wird. Das Verfahren umfasst die Schritte a) Anbringen eines Transdermalen Therapeutischen Systems enthaltend eine Rückschicht, eine den mindestens einen pharmazeutischen Wirkstoff enthaltende Reservoirschicht und ein Klebemittel auf eine bestimmte Stelle der Hautoberfläche dieser Person, b) Aufrechterhalten des Kontakts zwischen der den Wirkstoff freisetzenden Seite des Transdermalen Therapeutischen Systems und der bestimmten Stelle der Hautoberfläche über einen längeren Zeitraum und c) Entfernen des Transdermalen Therapeutischen Systems nach Ablauf des vorgesehenen Anwendungszeitraums, wobei der längere Zeitraum mindestens einen Zeitabschnitt umfasst, der mit einem intensiven Ausgesetztsein des auf der Hautoberfläche haftenden TTS mit Wasser verbunden ist.

Das Transdermale Therapeutische System zur Verabreichung mindestens eines pharmazeutischen Wirkstoffs durch die Haut einer Person, welche die kontinuierliche Verabreichung dieses Wirkstoffs über einen längeren Zeitraum benötigt, wobei dieser längere Zeitraum mindestens einen Zeitabschnitt intensiven Ausgesetztseins des Transdermalen Therapeutischen System mit Wasser enthält, umfaßt eine Rückschicht, eine Reservoirschicht und ein Klebemittel und besitzt die Neuerung, daß das Klebemittel seine klebenden Eigenschaften über den besagten längeren Zeitraum beibehält.

Die Abbildungen zeigen drei unterschiedliche Ausführungsformen für Konstruktionen von Transdermalen Therapeutischen Systemen (TTS), welche die in der Beschreibung genannten Konstruktionselemente enthalten. Dabei besitzen die Bezugszeichen folgende Bedeutung:
1 = Rückschicht
2 = Reservoirschicht
3 = Kontrollmembran
4 = Haftkleberschicht
5 = Trägerfolie

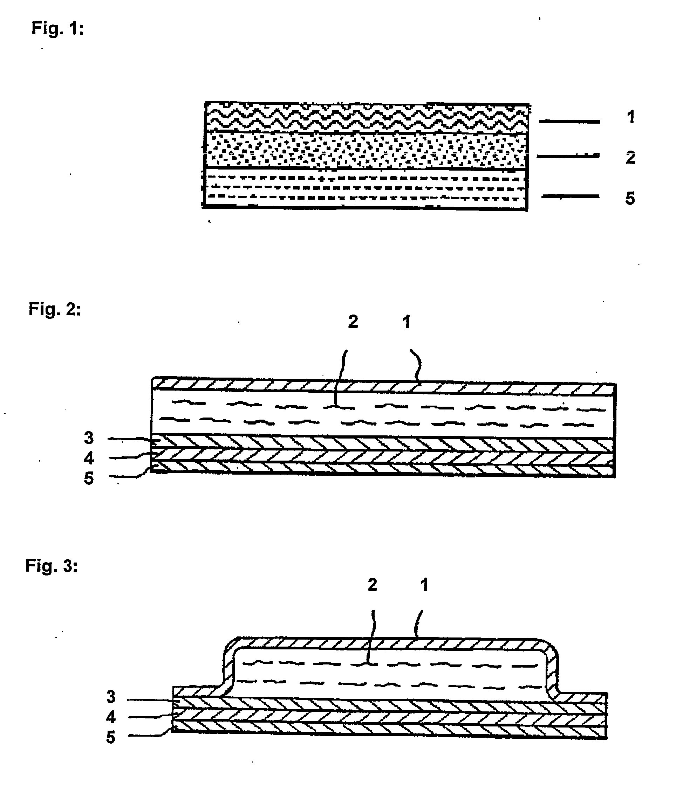

## Patentansprüche

1. Transdermales Therapeutisches System (TTS) zur kontinuierlichen Verabreichung mindestens eines pharmazeutischen Wirkstoffs durch die Haut einer Person, welche die kontinuierliche Verabreichung dieses Wirkstoffs über einen längeren Zeitraum benötigt und wobei während dieses längeren Zeitraums mindestens ein Zeitabschnitt auftritt, der mit einem intensiven Ausgesetztsein des auf der Hautoberfläche haftenden TTS mit Wasser verbunden ist, umfassend eine wirkstoffundurchlässige Rückschicht, die flexibel und durchlässig für Wasserdampf, jedoch für Wasser in flüssiger Form undurchlässig ist in Form einer Folienbahn, eine den mindestens einen pharmazeutischen Wirkstoff enthaltende Reservoirschicht, wobei dieser in der Reservoirschicht in einer solchen Menge enthalten ist, die ausreichend ist, dass er kontinuierlich über den längeren Zeitraum in einer wirksamen Menge transdermal an den Blutkreislauf abgegeben wird, und ein Klebemittel, welches seine klebenden Eigenschaften über den besagten längeren Zeitraum beibehält, **dadurch gekennzeichnet, dass** die Folienbahn perforiert ist und zwischen 200 und 2.500 Poren pro cm² aufweist, welche einen Durchmesser zwischen 2 und 150 µm besitzen.

2. Transdermales Therapeutisches System nach Anspruch 1, **dadurch gekennzeichnet, dass** die Rückschicht eine Folienbahn ist, die aus Kunststoff oder Metall besteht oder eines dieser Materialien im einem Verbundlaminat enthält und eine Dicke zwischen 2 und 50 µm, vorzugsweise zwischen 5 und 36 µm und besonders bevorzugt zwischen 9 und 23 µm aufweist.

3. Transdermales Therapeutisches System nach Anspruch 1, **dadurch gekennzeichnet, dass** die Rückschicht eine Folienbahn ist, die transparent oder undurchsichtig ist.

4. Transdermales Therapeutisches System nach einem der vorangegangenen Ansprüche, **dadurch gekennzeichnet, dass** die Porendichte zwischen 500 und 1.700 pro cm² liegt.

5. Transdermales Therapeutisches System nach einem der vorangegangenen Ansprüche, **dadurch gekennzeichnet, dass** die Poren eine trichterförmige Lochstruktur besitzen.

6. Transdermales Therapeutisches System nach einem der vorangegangenen Ansprüche, **dadurch gekennzeichnet, dass** die Folienbahn mit einem Gewebe kombiniert wird.

7. Transdermales Therapeutisches System nach einen oder mehreren der vorangehenden Ansprüche, **dadurch gekennzeichnet, dass** der Gehalt des Wirkstoffs in der Reservoirschicht zwischen 0,5 und 45 Gew.-%, vorzugsweise zwischen 3 und 25 Gew.-% liegt.

8. Transdermales Therapeutisches System nach einem oder mehreren der vorangehenden Ansprüche, **dadurch gekennzeichnet, dass** die Rückschicht elastisch ist und bei Anwendung einer äußeren Zugkraft in mindestens einer Richtung zu einer Streckung von mindestens 10%, vorzugsweise mindestens 30% befähigt ist, wobei bei Wegfall dieser Zugkraft die Rückschicht wieder die ursprünglichen Dimensionen annimmt.

9. Transdermales Therapeutisches System nach einem oder mehreren der vorangehenden Ansprüche, **dadurch gekennzeichnet, dass** der längere Zeitraum die Zeitspanne zwischen dem Anbringen des TTS auf der Hautoberfläche eines Anwenders und dem Abziehen des TTS nach Ablauf des vorgesehenen Anwendungszeitraums ist und mindestens 24 Stunden, vorzugsweise mindestens 72 Stunden beträgt und der innerhalb dieses Zeitraums einen Zeitabschnitt intensiven Ausgesetztseins des auf der Hautoberfläche haftenden TTS mit Wasser mit einer Dauer von mindestens 1 Minute bis maximal 30 Minuten, vorzugsweise 5 bis 15 Minuten umfasst.

## Claims

1. Transdermal therapeutic system (TTS) for continuous administration of at least one active pharmaceutical ingredient through the skin of a person requiring continuous administration of this active ingredient over a prolonged period, and where, during this prolonged period, at least one time segment associated with an intensive exposure to water of the TTS adhering to the surface of the skin occurs, comprising a backing layer which is impermeable to active ingredient and which is flexible and permeable to water vapour but impermeable to water in liquid form, in the form of a film web, a reservoir layer comprising the at least one active pharmaceutical ingredient, the latter being present in the reservoir layer in an amount sufficient for it to be delivered continuously over the prolonged period in an effective amount transdermally to the blood circulation, and an adhesive which retains its adhesive properties over the said prolonged period, **characterized in that** the film web is perforated and has between 200 and 2500 pores per cm² having a diameter of between 2 and 150 µm.

2. Transdermal therapeutic system according to Claim 1, **characterized in that** the backing layer is a film web which consists of plastic or metal or comprises one of these materials in a composite laminate and has a thickness of between 2 and 50 µm, preferably between 5 and 36 µm and particularly preferably between 9 and 23 µm.

3. Transdermal therapeutic system according to Claim 1, **characterized in that** the backing layer is a film web which is transparent or opaque.

4. Transdermal therapeutic system according to any of the preceding claims, **characterized in that** the pore density is between 500 and 1700 per cm².

5. Transdermal therapeutic system according to any of the preceding claims, **characterized in that** the pores have a funnel-shaped orifice structure.

6. Transdermal therapeutic system according to any of the preceding claims, **characterized in that** the film web is combined with a fabric.

7. Transdermal therapeutic system according to one or more of the preceding claims, **characterized in that** the content of the active ingredient in the reservoir layer is between 0.5 and 45% by weight, preferably between 3 and 25% by weight.

8. Transdermal therapeutic system according to one or more of the preceding claims, **characterized in that** the backing layer is elastic and, when an external tensile force is applied, is capable of stretching in at least one direction by at least 10%, preferably at least 30%, the backing layer returning to the original dimensions when this tensile force is removed.

9. Transdermal therapeutic system according to one or more of the preceding claims, **characterized in that** the prolonged period is the time between the attachment of the TTS to the surface of a user's skin and the removal of the TTS after the intended period of use, and is at least 24 hours, preferably at least 72 hours, and includes within this period a time segment of intensive exposure to water of the TTS adhering to the surface of the skin with a duration of at least 1 minute up to a maximum of 30 minutes, preferably 5 to 15 minutes.

## Revendications

1. Système thérapeutique transdermique (TTS, Transdermales Therapeutisches System) pour l'administration continue d'au moins une substance pharmaceutique active à travers la peau d'une personne, qui a besoin d'une administration continue de cette substance active pendant une très longue durée et dans lequel pendant cette très longue durée se produit au moins une coupure temporelle, qui est liée à une exposition intensive du TTS adhérant à la surface de la peau avec de l'eau, comprenant une couche dorsale perméable à la substance active, qui est flexible et perméable à la vapeur d'eau, cependant imperméable à l'eau sous forme liquide sous la forme d'un ruban de film, une couche de réservoir contenant au moins une substance pharmaceutique active, cette substance étant contenue dans la couche réservoir en une quantité, qui est suffisante pour qu'elle soit administrée en continu pendant la très longue durée en une quantité efficace par voie transdermique dans la circulation sanguine, et une colle, qui conserve ses caractéristiques collantes pendant la durée très longue indiquée, **caractérisé en ce que** le ruban de film est perforé et présente entre 200 et 2 500 pores par cm², qui possèdent un diamètre entre 2 et 150 µm.

2. Système thérapeutique transdermique selon la revendication 1, **caractérisé en ce que**, la couche dorsale est un ruban de film, qui est constitué de matière plastique ou de métal ou contient un de ces matériaux dans un stratifié composite et présente une épaisseur entre 2 et 50 µm, de préférence entre 5 et 36 µm et de manière particulièrement préférée entre 9 et 23 µm.

3. Système thérapeutique transdermique selon la revendication 1, **caractérisé en ce que**, la couche dorsale est un ruban de film, qui est transparent ou opaque.

4. Système thérapeutique transdermique selon une des revendications précédentes, **caractérisé en ce que**, la densité de pores se situe entre 500 et 1 700 par cm².

5. Système thérapeutique transdermique selon une des revendications précédentes, **caractérisé en ce que**, les pores possèdent une structure de trous en forme d'entonnoir.

6. Système thérapeutique transdermique selon une des revendications précédentes, **caractérisé en ce que**, le ruban de film est combiné avec un tissu.

7. Système thérapeutique transdermique selon une ou plusieurs des revendications précédentes, **caractérisé en ce que**, la teneur de la substance active dans la couche de réservoir se situe entre 0,5 et 45 % en poids, de préférence entre 3 et 25 % en poids.

8. Système thérapeutique transdermique selon une ou plusieurs des revendications précédentes, **caractérisé en ce que**, la couche dorsale est élastique et est apte si on utilise une force de traction externe dans au moins une direction à un allongement d'au moins 10 %, de préférence d'au moins 30 %, dans lequel si on supprime cette force de traction la couche dorsale reprend les dimensions d'origine.

9. Système thérapeutique transdermique selon une ou plusieurs des revendications précédentes, **caractérisé en ce que**, le temps très long est l'espace de temps entre le placement du TTS sur la surface de la peau d'un utilisateur et l'enlèvement du TTS après écoulement du temps d'utilisation prévu et s'élève à au moins 24 heures, de préférence au moins 72 heures et qui comprend à l'intérieur de cet espace de temps une coupure temporelle d'exposition intensive du TTS collé sur la surface de la peau à de l'eau pendant une durée d'au moins 1 minute jusqu'au maximum 30 minutes, de préférence de 5 à 15 minutes.
